# FASCICULE DE BREVET EUROPEEN

(11) **EP 1 885 705 B1**
(45) Date de publication et mention de la délivrance du brevet: **13.02.2019**
(21) Numéro de dépôt: 06755262.0
(22) Date de dépôt: 19.05.2006
(51) Int. Cl.: C07D 303/08, C07D 301/26, C07C 29/62, C07C 31/34

(54) **PROCÉDÉ DE FABRICATION D'UN ÉPOXYDE**
VERFAHREN ZUR HERSTELLUNG EINES EPOXIDS
METHOD FOR MAKING AN EPOXIDE

(30) Priorité: 20.05.2005 EP 05104321; 20.05.2005 FR 0505120; 08.11.2005 US 734635 P; 08.11.2005 US 734657 P; 08.11.2005 US 734636 P; 08.11.2005 US 734627 P; 08.11.2005 US 734634 P; 08.11.2005 US 734658 P; 08.11.2005 US 734637 P; 08.11.2005 US 734659 P
(43) Date de publication de la demande: 13.02.2008
(73) Titulaire: SOLVAY SA, 1120 Bruxelles (BE)
(72) Inventeur: GILBEAU, Patrick, B-7090 Braine-le-Comte (BE)
(74) Mandataire: Lavoix
(86) Numéro de dépôt international: PCT/EP2006/062437
(87) Numéro de publication internationale: WO 2006/100311

(56) Documents cités:
- EP-A- 0 561 441
- EP-A- 0 919 551
- WO-A-2005/021476
- WO-A-2005/054167
- GB-A- 467 481
- FAUCONNIER M A: "PREPARATION DE L'EPICHLORHYDRINE" CONFERENCE PROCEEDINGS ARTICLE, vol. 50, no. 50, 1888, pages 212-214, XP009046846

## Description

La présente invention concerne un procédé de fabrication d'épichlorhydrine. L'épichlorhydrine est une matière première importante pour la production d'autres composés. L'épichlorhydrine est une matière première importante pour la production de glycérol, de résines époxy, d'élastomères synthétiques, d'éthers de glycidyle, de résines polyamides, etc. (voir Ullmann's Encyclopedia of Industrial Chemistry, Fifth Edition, Vol. A9, p.539).

Dans la production industrielle de l'épichlorhydrine, la technologie la plus utilisée comprend les étapes suivantes : chloration substitutive radicalaire à haute température du propylène en chlorure d'allyle, hypochloration du chlorure d'allyle ainsi synthétisé en dichloropropanol et déshydrochloration du dichloropropanol en épichlorhydrine par une solution aqueuse alcaline. Une autre technologie utilisée à plus petite échelle comprend les étapes suivantes : acétoxylation catalytique du propylène en acétate d'allyle, hydrolyse de l'acétate d'allyle en alcool allylique, chloration catalytique de l'alcool allylique en dichloropropanol et déshydrochloration alcaline du dichloropropanol en épichlorhydrine. D'autres technologies qui n'ont pas encore reçu d'application industrielle peuvent être envisagées parmi lesquelles l'oxydation catalytique directe du chlorure d'allyle en épichlorhydrine au moyen de peroxyde d'hydrogène ou la chloration du glycérol en dichloropropanol suivie d'une déshydrochloration alcaline du dichloropropanol ainsi formé en épichlorhydrine, comme divulguée dans la demande de brevet WO2005021476.

Conformément à l'invention, on a découvert qu'un problème, notamment lorsque l'on met en oeuvre des chlorhydrines obtenues par chloration d'hydrocarbures aliphatiques poly hydroxylés dans une réaction de déshydrochloration, est la présence de cétones halogénées formées comme sous-produits. Ces cétones halogénées peuvent présenter des températures d'ébullition voisines de celles des époxydes et être difficilement séparables par une opération de distillation. Dans le cadre de l'invention, on a également découvert que les cétones halogénées, même à faible concentration, sont responsables du développement d'une coloration indésirable de l'épichlorhydrine ou des produits fabriqués au départ de celui-ci. C'est plus particulièrement le cas pour la chloroacétone formée dans le procédé de déshydrochloration du dichloropropanol pour former de l'épichlorhydrine.

On a aussi découvert que, de façon surprenante, on pouvait éliminer ces cétones lors de la fabrication de l'épichlorhydrine.

L'invention concerne donc un procédé de fabrication d'épichlorhydrine par déshydrochloration de dichloropropanol, dans lequel au moins une fraction du dichloropropanol est fabriquée par chloration de glycérol, et dans lequel de la chloroacétone est formée comme sous-produit et qui comprend au moins un traitement d'élimination d'au moins une partie de la chloroacétone formée.

L'expression « époxyde » est utilisée ici pour décrire un composé comportant au moins un oxygène ponté sur une liaison carbone-carbone. Généralement les atomes de carbone de la liaison carbone-carbone sont adjacents et le composé peut contenir d'autres atomes que des atomes de carbone et d'oxygène, tels que des atomes d'hydrogène et des halogènes. L'époxyde est l'épichlorhydrine.

L'expression « oléfine » est utilisée ici pour décrire un composé comportant au moins une liaison double carbone-carbone. Généralement le composé peut contenir d'autres atomes que des atomes de carbone, tels que des atomes d'hydrogène et des halogènes. Les oléfines préférées sont l'éthylène, le propylène, le chlorure d'allyle et les mélanges d'au moins deux d'entre-elles.

L'expression « hydrocarbure aliphatique poly hydroxylé » se rapporte à un hydrocarbure qui contient au moins deux groupements hydroxyles attachés à deux atomes de carbone différents saturés. L'hydrocarbure aliphatique poly hydroxylé peut contenir, mais n'est pas limité à, de 2 à 60 atomes de carbone.

Chacun des carbones d'un hydrocarbure aliphatique poly hydroxylé portant le groupement hydroxyl (OH) fonctionnel ne peut pas posséder plus d'un groupement OH, et doit être d'hybridation sp3. L'atome de carbone portant le groupement OH peut être primaire, secondaire ou tertiaire. L'hydrocarbure aliphatique poly hydroxylé utilise dans la présente invention doit contenir au moins deux atomes de carbone d'hybridation sp3 portant un groupement OH. L'hydrocarbure aliphatique poly hydroxylé inclut n'importe quel hydrocarbure contenant un diol vicinal (1,2-diol) ou un triol vicinal (1,2,3-triol) y compris des ordres plus élevés de ces unités répétitives, vicinales ou contiguës. La définition de l'hydrocarbure aliphatique poly hydroxylé inclut aussi par exemple un ou plus de groupements fonctionnels 1,3-, 1,4-, 1,5- et 1,6-diol. L'hydrocarbure aliphatique poly hydroxylé peut aussi être un polymère tel que l'alcool polyvinylique. Les diols géminés, par exemple, sont exclus de cette classe d'hydrocarbures aliphatiques poly hydroxylés.

Les hydrocarbures aliphatiques poly hydroxylés peuvent contenir des entités aromatiques ou des hétéro atomes incluant par exemple les hétéro atomes de type halogène, soufre, phosphore, azote, oxygène, silicium et bore, et leurs mélanges.

Des hydrocarbures aliphatiques poly hydroxylés utilisables dans la présente invention comprennent par exemple, le 1,2-éthanediol (éthylène glycol), le 1,2-propanediol (propylène glycol), le 1,3 -propanediol, le 1-chloro-2,3-propanediol (chloropropanediol), le 2-chloro-1,3-propanediol (chloropropanediol), le 1,4-butanediol, le 1,5-pentanediol, les cyclohexanediols, le 1,2-butanediol, le 1,2-cyclohexanediméthanol, le 1,2,3-propanetriol (aussi connu comme « glycérol » ou « glycérine »), et leurs mélanges. De façon préférée, l'hydrocarbure aliphatique poly hydroxylé utilisé dans la présente invention inclut par exemple le 1,2-éthanediol, le 1,2-propanediol, le 1,3-propanediol, le chloropropanediol et 1,2,3-propanetriol, et les mélanges d'au moins deux d'entre-eux. De façon plus préférée, l'hydrocarbure aliphatique poly hydroxylé utilisé dans la présente invention inclut par exemple le 1,2-éthanediol, le 1,2-propanediol, le chloropropanediol et 1,2,3-propanetriol, et les mélanges d'au moins deux d'entre-eux. Le 1,2,3-propanetriol ou glycérol est le plus préféré.

Les esters d'hydrocarbure aliphatique poly hydroxylé peuvent être présents dans l'hydrocarbure aliphatique poly hydroxylé et/ou être produits dans le procédé de fabrication de la chlorhydrine et/ou être fabriqués préalablement au procédé de fabrication de la chlorhydrine. Des exemples d'esters de l'hydrocarbure aliphatique poly hydroxylé comprennent le monoacétate de l'éthylène glycol, les monoacétates de propanediol, les monoacétates de glycérol, les monostéarates de glycérol, les diacétates de glycérol et leurs mélanges.

Dans le procédé selon l'invention, les esters de l'hydrocarbure aliphatique polyhydroxylé peuvent provenir de la réaction entre l'hydrocarbure aliphatique polyhydroxylé et un acide organique, avant, pendant ou dans les étapes qui suivent la réaction avec l'agent de chloration.

L'expression « chorhydrine » est ici utilisée pour décrire un composé contenant au moins un groupement hydroxyle et au moins un atome de chlore attaché à différents atomes de carbone saturés. La chorhydrine est le dichloropropanol.

La chlorhydrine dans le procédé selon l'invention peut être obtenue au départ de matières premières fossiles ou au départ de matières premières renouvelables, de préférence au départ de matières premières renouvelables.

Par matières premières fossiles, on entend désigner des matières issues du traitement des ressources naturelles pétrochimiques, par exemple le pétrole, le gaz naturel, et le charbon. Parmi ces matières, les composés organiques comportant 2 et 3 atomes de carbone sont préférés. La chlorhydrine étant le dichloropropanol, le chlorure d'allyle, l'alcool allylique et le glycérol « synthétique » sont particulièrement préférés. Par glycérol « synthétique », on entend désigner un glycérol généralement obtenu à partir de ressources pétrochimiques.

Par matières premières renouvelables, on entend désigner des matières issues du traitement des ressources naturelles renouvelables. Parmi ces matières, l'éthylène glycol « naturel », le propylène glycol « naturel » et le glycérol « naturel » sont préférés. De l'éthylène glycol, du propylène glycol et du glycérol « naturels » sont par exemple obtenus par conversion de sucres via des procédés thermochimiques, ces sucres pouvant être obtenus au départ de biomasse, comme décrit dans "Industrial Bioproducts : Today and Tomorrow, Energetics, Incorporated for the US. Department of Energy, Office of Energy Efficiency and Renewable Energy, Office of the Biomass Program, July 2003, pages 49, 52 to 56". Un de ces procédés est par exemple l'hydrogénolyse catalytique du sorbitol obtenu par conversion thermochimique du glucose. Un autre procédé est par exemple l'hydrogénolyse catalytique du xylitol obtenu par hydrogénation du xylose. Le xylose peut par exemple être obtenu par hydrolyse de l'hemicellulose contenue dans les fibres de maïs. Par « glycérol naturel » ou par « glycérol obtenu à partir de matières premières renouvelables », on entend désigner en particulier du glycérol obtenu au cours de la fabrication de biodiesel ou encore du glycérol obtenu au cours de transformations de graisses ou huiles d'origine végétale ou animale en général telles que des réactions de saponification, de transestérification ou d'hydrolyse.

Parmi les huiles utilisables pour fabriquer le glycérol naturel, on peut citer toutes les huiles courantes, comme les huiles de palme, de palmiste, de coprah, de babassu, de colza ancien ou nouveau, de tournesol, de mais, de ricin et de coton, les huiles d'arachide, de soja, de lin et de crambe et toutes les huiles issues par exemple des plantes de tournesol ou de colza obtenues par modification génétique ou hybridation.

On peut même utiliser des huiles de friture usagées, des huiles animales variées, comme les huiles de poisson, le suif, le saindoux et même des graisses d'équarrissage.

Parmi les huiles utilisées, on peut encore indiquer des huiles partiellement modifiées par exemple par polymérisation ou oligomérisation comme par exemple les "standolies" d'huiles de lin, de tournesol et les huiles végétales soufflées.

Un glycérol particulièrement adapté peut être obtenu lors de la transformation de graisses animales. Un autre glycérol particulièrement adapté peut être obtenu lors de la fabrication de biodiesel. Un troisième glycérol tout particulièrement bien adapté peut être obtenu lors de la transformation de graisses ou d'huiles, animales ou végétales, par trans-estérification en présence d'un catalyseur hétérogène, tel que décrit dans les documents FR 2752242, FR 2869612 et FR 2869613. Plus spécifiquement, le catalyseur hétérogène est choisi parmi les oxydes mixtes d'aluminium et de zinc, les oxydes mixtes de zinc et de titane, les oxydes mixtes de zinc, de titane et d'aluminium, et les oxydes mixtes de bismuth et d'aluminium, et le catalyseur hétérogène est mis en oeuvre sous la forme d'un lit fixe. Ce dernier procédé peut être un procédé de fabrication de biodiesel.

Le dichloropropanol peut être obtenu au départ de ces matières premières par n'importe quel procédé. Les procédés d'hypochloration du chlorure d'allyle, de chloration de l'alcool allylique et de chloration du glycérol « synthétique » et/ou « naturel » sont préférés. Le procédé de chloration du glycérol « synthétique » et/ou « naturel » est particulièrement préféré.

Dans le procédé de fabrication de l'épichlorhydrine selon l'invention, au moins une fraction du dichloropropanol est fabriquée par chloration du glycérol. Le glycérol peut être « synthétique » ou « naturel » aux sens définis ci-dessus.

Dans le procédé de fabrication selon l'invention, le glycérol « naturel » c'est-à-dire obtenu au cours de la fabrication de biodiesel, ou au cours de transformations de graisses ou huiles, d'origine végétale ou animale, les transformations étant sélectionnées parmi les réactions de saponification, de trans-estérification et d'hydrolyse, est préféré. Le glycérol obtenu par trans-estérification de graisses ou huiles, d'origine végétale ou animale, et dans lequel la trans-estérification est réalisée en présence d'un catalyseur hétérogène, est particulièrement préféré. Dans le procédé de fabrication de l'épichlorhydrine selon l'invention, le hydrocarbure aliphatique polyhydroxylé peut être tel que décrit dans la demande de brevet « Procédé de préparation de chlorhydrine par conversion d'hydrocarbures aliphatiques poly hydroxylés » déposée au nom de SOLVAY SA le même jour que la présente demande.

Mention particulière est faite d'un procédé de fabrication d'une chlorhydrine dans lequel on fait réagir un hydrocarbure aliphatique poly hydroxylé, un ester d'un hydrocarbure aliphatique poly hydroxylé, ou un mélange d'entre eux, dont la teneur totale en métaux exprimés sous forme d'éléments est supérieure ou égale à 0,1 µg/kg et inférieure ou égale à 1 000 mg/kg, avec un agent de chloration.

Un premier avantage lié à ce dernier type de glycérol est qu'il ne contient pas ou peu de métaux. Ces métaux peuvent être gênants dans certaines étapes de la fabrication du dichloropropanol, comme par exemple des étapes de traitement de résidus. Un second avantage lié à ce dernier type de glycérol est qu'il ne contient pas ou peu de composés organiques lourds qui peuvent s'accumuler dans la fabrication du dichloropropanol. Les opérations de purge destinées à éliminer ces produits organiques lourds peuvent en conséquence être réduites.

Dans le procédé de fabrication de l'épichlorhydrine selon l'invention, la mise en oeuvre de la chlorhydrine obtenue au départ de l'hydrocarbure aliphatique poly hydroxylé par réaction avec un agent de chloration, peut être effectuée par exemple selon le procédé décrit dans la demande WO 2005/054167 de SOLVAY SA.

Dans le procédé de fabrication de l'épichlorhydrine selon l'invention, l'hydrocarbure aliphatique poly hydroxylé peut être un produit brut ou un produit épuré tel que décrit dans la demande WO 2005/054167 de SOLVAY SA, de la page 2, ligne 8, à la page 4, ligne 2.

Le produit brut peut contenir des acides gras, des esters d'acides gras tels qu'en particulier des monoglycérides ou des diglycérides, éventuellement en combinaison avec de l'eau et/ou un sel de métal. On préfère utiliser un glycérol épuré c'est-à-dire contenant au moins 80 % et au plus 99,9 % en poids de glycérol, au moins 0,1 % et au plus 20 % en poids d'eau, au moins 1 mg/kg et au plus 0,1 % en poids d'aldéhyde et au moins 10 mg/kg et au plus 10 % en poids de méthanol et/ou d'éthanol.

Dans le procédé de fabrication de l'épichlorhydrine selon l'invention, l'hydrocarbure aliphatique poly hydroxylé peut être un hydrocarbure aliphatique poly hydroxylé dont la teneur en métaux alcalin et/ou alcalino-terreux est inférieure ou égale à 5 g/kg tel que décrit dans la demande intitulée « Procédé de fabrication d'une chlorhydrine par chloration d'un hydrocarbure aliphatique poly hydroxylé » déposée au nom de SOLVAY SA le même jour que la présente demande.

Dans le procédé selon l'invention, la teneur en métaux alcalins et/ou alcalino-terreux de l'hydrocarbure aliphatique poly hydroxylé, de l'ester d'un hydrocarbure aliphatique poly hydroxylé, ou du mélange d'entre eux, est inférieure ou égale à 5 g/kg, souvent inférieure ou égale à 1 g/kg, plus particulièrement inférieure ou égale à 0,01 g/kg et dans certains cas inférieure ou égale à 2 mg/kg. La teneur métaux alcalins et/ou alcalino-terreux de l'hydrocarbure aliphatique poly hydroxylé est généralement supérieure ou égale à 0,1 µg/kg.

Dans le procédé selon l'invention, les métaux alcalins sont généralement le lithium, le sodium, le potassium et le césium, souvent le sodium et le potassium, et fréquemment le sodium.

Dans le procédé de fabrication d'une chlorhydrine, la teneur en lithium de l'hydrocarbure aliphatique poly hydroxylé, de l'ester d'hydrocarbure aliphatique poly hydroxylé, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, la teneur en sodium de l'hydrocarbure aliphatique poly hydroxylé, de l'ester d'hydrocarbure aliphatique poly hydroxylé, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, la teneur en potassium de l'hydrocarbure aliphatique poly hydroxylé, de l'ester d'hydrocarbure aliphatique poly hydroxylé, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, la teneur en rubidium de l'hydrocarbure aliphatique poly hydroxylé, de l'ester d'hydrocarbure aliphatique poly hydroxylé, ou du mélange d'entre eux, est généralement inférieure ou égale à 1 g/kg, souvent inférieure ou égale à 0,1 g/kg et plus particulièrement inférieure ou égale à 2mg/kg. Cette teneur est généralement supérieure ou égale à 0,1 µg/kg.

Dans le procédé selon l'invention, la teneur en césium de l'hydrocarbure aliphatique poly hydroxylé, de l'ester d'hydrocarbure aliphatique poly hydroxylé, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, les éléments alcalino-terreux sont généralement le magnésium, le calcium, le strontium et le barium, souvent le magnésium et le calcium et fréquemment le calcium.

Dans le procédé selon l'invention, la teneur en magnésium de l'hydrocarbure aliphatique poly hydroxylé, de l'ester d'hydrocarbure aliphatique poly hydroxylé, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, la teneur en calcium de l'hydrocarbure aliphatique poly hydroxylé, de l'ester d'hydrocarbure aliphatique poly hydroxylé, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, la teneur en strontium de l'hydrocarbure aliphatique poly hydroxylé, de l'ester d'hydrocarbure aliphatique poly hydroxylé, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, la teneur en barium de l'hydrocarbure aliphatique poly hydroxylé, de l'ester d'hydrocarbure aliphatique poly hydroxylé, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, les métaux alcalins et/ou alcalinoterreux sont généralement présents sous la forme de sels, fréquemment sous la forme de chlorures, de sulfates et de leurs mélanges. Le chlorure de sodium est le plus souvent rencontré.

Dans le procédé de fabrication de l'épichlorhydrine selon l'invention, l'agent de chloration de l'hydrocarbure aliphatique poly hydroxylé peut être le chlorure d'hydrogène et/ou l'acide chlorhydrique tels que décrits dans la demande WO 2005/054167 de SOLVAY SA, de la page 4, ligne 30, à la page 6, ligne 2.

Le chlorure d'hydrogène peut provenir d'un procédé de pyrolyse de composés organiques chlorés comme par exemple d'une fabrication de chlorure de vinyle, d'un procédé de fabrication de 4,4-méthylènediphenyl di-isocyanate (MDI) ou de toluène di-isocyanate (TDI), de procédés de décapage des métaux ou d'une réaction entre un acide inorganique comme l'acide sulfurique ou phosphorique et un chlorure métallique tel que le chlorure de sodium, le chlorure de potassium ou le chlorure de calcium.

Dans un mode de réalisation avantageux du procédé de fabrication de l'épichlorhydrine selon l'invention, l'agent de chloration de l'hydrocarbure aliphatique poly hydroxylé est du chlorure d'hydrogène gazeux ou une solution aqueuse de chlorure d'hydrogène ou une combinaison des deux.

Dans le procédé de fabrication de l'épichlorhydrine selon l'invention, l'agent de
chloration de l'hydrocarbure aliphatique poly hydroxylé, peut être de l'acide chlorhydrique aqueux ou du chlorure d'hydrogène de préférence anhydre, issu d'un procédé de fabrication de chlorure d'allyle et/ou de chlorométhanes et/ou de chlorinolyse et/ou d'oxydation à haute température de composés chlorés tels que décrit dans la demande intitulée « Procédé de fabrication d'une chlorhydrine par réaction entre un hydrocarbure aliphatique poly hydroxylé et un agent de chloration » déposée au nom de SOLVAY SA le même jour que la présente demande.

Mention particulière est faite d'un procédé de fabrication d'une chlorhydrine à partir d'un hydrocarbure aliphatique poly hydroxylé, d'un ester d'un hydrocarbure aliphatique poly hydroxylé, ou d'un mélange d'entre eux, et d'un agent de chloration, ce dernier contenant au moins un des composés suivants : azote, oxygène, hydrogène, chlore, un composé organique hydrocarboné, un composé organique halogéné, un composé organique oxygéné et un metal.

Mention particulière est faite d'un composé organique hydrocarboné est choisi parmi les hydrocarbures aromatiques, aliphatiques saturés ou insaturés et leurs mélanges.

Mention particulière est faite d'un hydrocarbure aliphatique insaturé qui est choisi parmi l'acétylène, l'éthylène, le propylène, le butène, le propadiène, le méthylacétylène, et leurs mélanges, d'un hydrocarbure aliphatique saturé qui est choisi parmi le méthane, l'éthane, le propane, le butane, et leurs mélanges, et d'un hydrocarbure aromatique qui est le benzène.

Mention particulière est faite d'un composé organique halogéné qui est un composé organique chloré choisi parmi les chlorométhanes, les chloroéthanes, les chloropropanes, les chlorobutanes, le chlorure de vinyle, le chlorure de vinylidène, les monochloropropènes, le perchloroéthylène, le trichloréthylène, les chlorobutadiène, les chlorobenzènes et leurs mélanges.

Mention particulière est faite d'un composé organique halogéné qui est un composé organique fluoré choisi parmi les fluorométhanes, les fluoroéthanes, le fluorure de Vinyle, le fluorure de vinylidène, et leurs mélanges.

Mention particulière est faite d'un composé organique oxygéné qui est choisi parmi les alcools, les chloroalcools, les chloroéthers et leurs mélanges

Mention particulière est faite d'un métal choisi parmi les métaux alcalins, les métaux alcalino-terreux, le fer, le nickel, le cuivre, le plomb, l'arsenic, le cobalt, le titane, le cadmium, l'antimoine, le mercure, le zinc, le sélénium, l'aluminium, le bismuth, et leurs mélanges.

Mention est plus particulièrement faite d'un procédé dans lequel l'agent de chloration est issu au moins partiellement d'un procédé de fabrication de chlorure d'allyle et/ou d'un procédé de fabrication de chlorométhanes et/ou d'un procédé de chlorinolyse et/ou d'un procédé d'oxydation de composés chlorés à une température supérieure ou égale à 800°C.

Dans un mode de réalisation avantageux du procédé de fabrication de l'épichlorhydrine selon l'invention, l'agent de chloration de l'hydrocarbure aliphatique poly hydroxylé ne contient pas de chlorure d'hydrogène gazeux.

Dans le procédé de fabrication de l'épichlorhydrine selon l'invention, la réaction de chloration de l'hydrocarbure aliphatique poly hydroxylé, peut être effectuée dans un réacteur tel que décrit dans la demande WO 2005/054167 de SOLVAY SA, à la page 6, lignes 3 à 23.

Mention est particulièrement faite d'une installation réalisée en, ou recouverte de, matériaux résistants dans les conditions de la réaction aux agents de chloration, en particulier au chlorure d'hydrogène. Mention est plus particulièrement faite d'une installation réalisée en acier émaillé ou en tantale.

Dans le procédé de fabrication de l'épichlorhydrine selon l'invention, la réaction de chloration de l'hydrocarbure aliphatique poly hydroxylé, de l'ester d'hydrocarbure aliphatique poly hydroxylé, ou du mélange d'entre eux, peut être effectuée dans des équipements, réalisés en ou recouverts de, matériaux résistant aux agents de chloration, tels que décrit dans la demande intitulée « Procédé de fabrication d'une chlorhydrine dans des équipements résistant à la corrosion » déposée au nom de SOLVAY SA le même jour que la présente demande.

Mention particulière est faite d'un procédé de fabrication d'une chlorhydrine comprenant une étape dans laquelle on soumet un hydrocarbure aliphatique poly hydroxylé, un ester d'hydrocarbure aliphatique poly hydroxylé, ou un mélange d'entre eux, à une réaction avec un agent de chloration contenant du chlorure d'hydrogène, et au moins une autre étape effectuée dans un équipement, réalisé en ou recouvert de, matériaux résistant à l'agent de chloration, dans les conditions de réalisation de cette étape. Mention est plus particulièrement faite de matériaux métalliques tels que l'acier émaillé, l'or et le tantale et de matériaux non-métalliques tels que le polyéthylène haute densité, le polypropylène, le poly(fluorure-de-vinylidène), le polytétrafluoroéthylène, les perfluoro alcoxyalcanes et le poly(perfluoropropylvinyléther), les polysulfones et les polysulfures, le graphite et le graphite imprégné.

Dans le procédé de fabrication de l'épichlorhydrine selon l'invention, la réaction de chloration de l'hydrocarbure aliphatique poly hydroxylé, de l'ester d'hydrocarbure aliphatique poly hydroxylé, ou du mélange d'entre eux, peut être effectuée dans un milieu réactionnel, tel que décrit dans la demande intitulée « Procédé continu de fabrication de chlorhydrines » déposée au nom de SOLVAY SA le même jour que la présente demande.

Mention particulière est faite d'un procédé continu de production de chlorhydrine dans lequel on fait réagir un hydrocarbure aliphatique poly hydroxylé, un ester d'un hydrocarbure aliphatique poly hydroxylé ou un mélange d'entre eux, avec un agent de chloration et un acide organique dans un milieu réactionnel liquide dont la composition à l'état stationnaire comprend de l'hydrocarbure aliphatique poly hydroxylé et des esters de l'hydrocarbure aliphatique poly hydroxylé dont la somme des teneurs exprimée en mole d'hydrocarbure aliphatique poly hydroxylé est supérieure à 1,1 mol % et inférieure ou égale à 30 mol %, le pourcentage étant rapporté à la partie organique du milieu réactionnel liquide.

La partie organique du milieu réactionnel liquide consiste en l'ensemble des composés organiques du milieu réactionnel liquide c'est-à-dire les composés dont la molécule contient au moins 1 atome de carbone.

Dans le procédé de fabrication de l'épichlorhydrine selon l'invention, la réaction de chloration de l'hydrocarbure aliphatique poly hydroxylé, peut être effectuée en présence d'un catalyseur tel que décrit dans la demande WO 2005/054167 de SOLVAY SA, de la page 6, ligne 28, à la page 8, ligne 5.

Mention est particulièrement faite d'un catalyseur basé sur un acide carboxylique ou sur un dérivé d'acide carboxylique ayant un point d'ébullition atmosphérique supérieur ou égal à 200°C, en particulier l'acide adipique et les dérivés de l'acide adipique.

Dans le procédé de fabrication de l'épichlorhydrine selon l'invention, la réaction de chloration de l'hydrocarbure aliphatique poly hydroxylé peut être effectuée à une concentration en catalyseur, une température, à une pression et pour des temps de séjour tels que décrits dans la demande WO 2005/054167 de SOLVAY SA, de la page 8, ligne 6 à la page 10, ligne 10.

Mention est particulièrement faite d'une température d'au moins 20°C et d'au plus 160°C, d'une pression d'au moins 0,3 bar et d'au plus, 100 bar, et d'un temps de séjour d'au moins 1 h et d'au plus 50 h.

Dans le procédé de fabrication de l'épichlorhydrine selon l'invention, la réaction de chloration de l'hydrocarbure aliphatique poly hydroxylé peut être effectuée en présence d'un solvant tel que décrit dans la demande WO 2005/054167 de SOLVAY SA, à la page 11, lignes 12 à 36.

Mention est particulièrement faite d'un solvant organique tel qu'un solvant organique chloré, un alcool, une cétone, un ester ou un éther, un solvant non aqueux miscible avec l'hydrocarbure aliphatique polyhydroxylé tel que le chlroéthanol, le chloropropanol, le chloropropanediol, le dichloropropanol, le dioxanne, le phénol, le crésol, et les mélanges de chloropropanediol et de dichloropropanol, ou des produits lourds de la réaction tels que les oligomères de l'hydrocarbure aliphatique poly hydroxylé au moins partiellement chlorés et/ou estérifiés.

Dans le procédé de fabrication de l'épichlorhydrine selon l'invention, la réaction de chloration de l'hydrocarbure aliphatique poly hydroxylé peut être effectuée en présence d'une phase liquide comprenant des composés lourds autres que l'hydrocarbure aliphatique poly hydroxylé, comme décrit dans la demande intitulée « Procédé de fabrication d'une chlorhydrine dans une phase liquide » déposée au nom de SOLVAY SA le même jour que la présente demande.

Mention particulière est faite d'un procédé de fabrication d'une chlorhydrine, dans lequel on soumet un hydrocarbure aliphatique poly hydroxylé, un ester d'hydrocarbure aliphatique poly hydroxylé, ou un mélange d'entre eux, à une réaction avec un agent de chloration, en présence d'une phase liquide comprenant des composés lourds autres que l'hydrocarbure aliphatique poly hydroxylé et dont la température d'ébullition sous une pression de 1 bar absolu est d'au moins 15 °C supérieure à la température d'ébullition de la chlorhydrine sous une pression de 1 bar absolu.

Dans le procédé de fabrication de l'épichlorhydrine selon l'invention, la réaction de chloration de l'hydrocarbure aliphatique poly hydroxylé, de l'ester d'hydrocarbure aliphatique poly hydroxylé, ou du mélange d'entre eux, peut être effectuée en mode batch ou en mode continu. Le mode continu est particulièrement préféré.

Dans le procédé de fabrication de l'épichlorhydrine selon l'invention, la réaction de chloration de l'hydrocarbure aliphatique poly hydroxylé, est préférentiellement effectuée dans un milieu réactionnel liquide. Le milieu réactionnel liquide peut être mono- ou multiphasique.

Le milieu réactionnel liquide est constitué par l'ensemble des composés solides dissous ou disperses, liquides dissous ou disperses et gazeux dissous ou disperses, à la température de la réaction.

Le milieu réactionnel comprend les réactifs, le catalyseur, le solvant, les impuretés présentes dans les réactifs, dans le solvant et dans le catalyseur, les intermédiaires de réaction, les produits et les sous-produits de la réaction.

Par réactifs, on entend désigner l'hydrocarbure aliphatique poly hydroxylé, l'ester d'hydrocarbure aliphatique poly hydroxylé et l'agent de chloration.

Parmi les impuretés présentes dans l'hydrocarbure aliphatique poly hydroxylé, on peut citer les acides carboxyliques, les sels d'acides carboxyliques, les esters d'acide gras avec l'hydrocarbure aliphatique poly hydroxylé, les esters d'acides gras avec les alcools utilisés lors de la trans-estérification, les sels inorganiques tels que les chlorures et les sulfates alcalins ou alcalino-terreux.

Lorsque l'hydrocarbure aliphatique poly hydroxylé est le glycérol, on peut citer parmi les impuretés du glycérol les acides carboxyliques, les sels d'acides carboxyliques, les esters d'acide gras tels que les mono-, les di- et les triglycérides, les esters d'acides gras avec les alcools utilises lors de la transestérification, les sels inorganiques tels que les chlorures et les sulfates alcalins ou alcalino -terreux.

Parmi les intermédiaires réactionnels on peut citer les monochlorhydrines de l'hydrocarbure aliphatique poly hydroxylé et leurs esters et/ou polyesters, les esters et/ou polyesters de l'hydrocarbure aliphatique poly hydroxylé et les esters des polychlorhydrines.

La chlorhydrine étant le dichloropropanol, on peut citer parmi les intermédiaires réactionnels, la monochlorhydrine de glycérol et ses esters et/ou polyesters, les esters et/ou polyesters de glycérol et les esters de dichloropropanol.

L'ester d'hydrocarbure aliphatique poly hydroxylé peut donc être selon le cas, un réactif, une impureté de l'hydrocarbure aliphatique poly hydroxylé ou un intermédiaire réactionnel.

Par produits de la réaction, on entend désigner la chlorhydrine et l'eau. L'eau peut être l'eau formée dans la réaction de chloration et/ou étre de l'eau introduite dans le procédé, par exemple via l'hydrocarbure aliphatique poly hydroxylé et/ou l'agent de chloration, tel que décrit dans la demande WO 2005/054167 de SOLVAY SA, à la page 2, lignes 22 à 28, à la page 3, lignes 20 à 25, à la page 5,lignes 7 à 31 et à la page 12, lignes 14 à 19.

Parmi les sous-produits, on peut citer par exemple, les oligomères l'hydrocarbure aliphatique poly hydroxylé partiellement chlorés et/ou estérifiés.

Lorsque l'hydrocarbure aliphatique poly hydroxylé est le glycérol, parmi les sous-produits, on peut citer par exemple, les oligomères du glycérol partiellement chlorés et/ou estérifiés.

Les intermédiaires réactionnels et les sous-produits peuvent être formés dans les différentes étapes du procédé comme par exemple, au cours de l'étape de fabrication de la chlorhydrine et au cours des étapes de séparation de la chlorhydrine.

Le milieu réactionnel liquide peut ainsi contenir l'hydrocarbure aliphatique poly hydroxylé, l'agent de chloration dissous ou dispersé sous forme de bulles, le catalyseur, le solvant, les impuretés présentes dans les réactifs, le solvant et le catalyseur, comme des sels dissous ou solides par exemple, les intermédiaires réactionnels, les produits et les sous-produits de la réaction.

Dans le procédé selon l'invention, la séparation de la chlorhydrine et des autres composés du milieu réactionnel, peut être effectuée selon les modes tels que décrits dans la demande WO 2005/054167 de SOLVAY SA, de la page 12, ligne 1, à la page 16, ligne 35 et à la page 18, lignes 6 à 13. Mention particulière est faite d'une séparation par distillation azéotropique d'un mélange eau/chlorhydrine/agent de chloration dans des conditions minimisant les pertes en agent de chloration suivie d'une séparation de la chlorhydrine par décantation. Ces autres composés sont ceux mentionnés ci-dessus et comprennent les réactifs non consommés, les impuretés présentes dans les réactifs, le catalyseur et le solvant, le solvant, le catalyseur, les intermédiaires réactionnels, l'eau et les sous produits de la réaction.

Dans le procédé de fabrication de l'épichlorhydrine selon l'invention, la séparation de la chlorhydrine et des autres composés du milieu réactionnel de chloration de l'hydrocarbure aliphatique polyhydroxylé, peut être effectuée selon des modes tels que décrits dans la demande de brevet EP 05104321.4 déposée au nom de SOLVAY SA le 20 mai 2005.

Mention particulière est faite d'un procédé de fabrication d'une chlorhydrine par réaction entre un hydrocarbure aliphatique poly hydroxylé, un ester d'un hydrocarbure aliphatique poly hydroxylé ou un mélange d'entre eux, et un agent de chloration dans lequel l'hydrocarbure aliphatique poly hydroxylé, un ester d'un hydrocarbure aliphatique poly hydroxylé ou un mélange d'entre eux, utilise contient au moins un sel métallique solide ou dissous, le procédé comprenant une opération de séparation destinée à enlever une partie du sel métallique. Mention est plus particulièrement est faite d'un procédé de fabrication d'une chlorhydrine par réaction entre un hydrocarbure aliphatique poly hydroxylé, un ester d'un hydrocarbure aliphatique poly hydroxylé ou un mélange d'entre eux, et un agent de chloration dans lequel l'hydrocarbure aliphatique poly hydroxylé, un ester d'un hydrocarbure aliphatique poly hydroxylé ou un mélange d'entre eux, utilise contient au moins un chlorure et/ou un sulfate de sodium et/ou potassium et dans lequel l'opération de séparation destinée à enlever une partie du sel métallique est un opération de filtration. Mention est aussi particulièrement faite d'un procédé de fabrication d'une chlorhydrine dans lequel (a) on soumet un hydrocarbure aliphatique poly hydroxylé, un ester d'un hydrocarbure aliphatique poly hydroxylé ou un mélange d'entre eux, à une réaction avec un agent de chloration dans un milieu réactionnel, (b) on prélève en continu ou périodiquement une fraction du milieu réactionnel contenant au moins de l'eau et la chlorhydrine, (c) au moins une partie de la fraction obtenue à l'étape (b) est introduite dans une étape de distillation et (d) le taux de reflux de l'étape de distillation est contrôlé en fournissant de l'eau à ladite étape de distillation. Mention est tout particulièrement faite d'un procédé de fabrication d'une chlorhydrine dans lequel (a) on soumet un hydrocarbure aliphatique poly hydroxylé, un ester d'un hydrocarbure aliphatique poly hydroxylé ou un mélange d'entre eux, à une réaction avec du chlorure d'hydrogène dans un milieu réactionnel, (b) on prélève en continu ou périodiquement une fraction du milieu réactionnel contenant au moins de l'eau et la chlorhydrine, (c) au moins une partie de la fraction obtenue à l'étape (b) est introduite dans une étape de distillation, dans lequel le rapport entre la concentration en chlorure d'hydrogène et la concentration en eau dans la fraction introduite dans l'étape de distillation est plus petit que le rapport de concentrations chlorure d'hydrogène/eau dans la composition binaire azéotropique chlorure d'hydrogène/eau à la température et à la pression de distillation.

Dans le procédé de fabrication de l'épichlorhydrine selon l'invention, la séparation de la chlorhydrine et des autres composés du milieu réactionnel de chloration de l'hydrocarbure aliphatique polyhydroxylé, de l'ester d'hydrocarbure aliphatique polyhydroxylé, ou des mélanges d'entre eux, peut être effectuée selon les modes tels que décrits dans la demande intitulée « Procédé de fabrication d'une chlorhydrine » déposée au nom de SOLVAY SA, le même jour que la présente demande.

Mention particulière est faite d'un procédé de fabrication d'une chlorhydrine comprenant les étapes suivantes : (a) on fait réagir un hydrocarbure aliphatique polyhydroxylé, un ester d'un hydrocarbure aliphatique polyhydroxylé, ou un mélange d'entre eux, avec un agent de chloration et un acide organique de façon à obtenir un mélange contenant de la chlorhydrine et des esters de la chlorhydrine, (b) on soumet au moins une partie du mélange obtenu à l'étape (a) à un ou plusieurs traitements dans des étapes ultérieures à l'étape (a) et (c) on ajoute de l'hydrocarbure aliphatique polyhydroxylé à au moins une des étapes ultérieures à l'étape (a), pour qu'il réagisse, à une température supérieure ou égale à 20 °C, avec les esters de la chlorhydrine de façon à former au moins partiellement des esters de l'hydrocarbure aliphatique polyhydroxylé.

Mention est plus particulièrement faite d'un procédé dans lequel l'hydrocarbure aliphatique polyhydroxylé est le glycérol et la chlorhydrine est le dichloropropanol.

Dans le procédé de fabrication de l'épichlorhydrine selon l'invention, la séparation de la chlorhydrine et des autres composés du milieu réactionnel de chloration de l'hydrocarbure aliphatique polyhydroxylé, de l'ester d'hydrocarbure poly hydroxylé, ou du mélange d'entre eux, peut être effectuée selon les modes tels que décrits dans la demande intitulée « Procédé de fabrication d'une chlorhydrine au départ d'un hydrocarbure aliphatique poly hydroxylé » déposée au nom de SOLVAY SA le même jour que la présente demande.

Mention particulière est faite d'un procédé de fabrication de chlorhydrine par réaction entre un hydrocarbure aliphatique poly hydroxylé, un ester d'hydrocarbure poly hydroxylé, ou un mélange d'entre eux, et un agent de chloration dans un réacteur qui est alimenté en un ou plusieurs flux liquides contenant moins de 50 % en poids de l'hydrocarbure aliphatique poly hydroxylé, de l'ester d'hydrocarbure poly hydroxylé, ou du mélange d'entre eux, par rapport au poids de la totalité des flux liquides introduits dans le réacteur. Mention plus particulière est faite d'un procédé comprenant les étapes suivantes : (a) on fait réagir un hydrocarbure aliphatique poly hydroxylé, un ester d'hydrocarbure poly hydroxylé, ou un mélange d'entre eux, avec un agent de chloration de façon à obtenir au moins un milieu contenant du de la chlorhydrine, de l'eau et de l'agent de chloration, (b) on prélève au moins une fraction du milieu formé à l'étape (a) et (c) on soumet la fraction prélevée à l'étape (b) à une opération de distillation et/ou de stripping dans laquelle on ajoute de l'hydrocarbure aliphatique poly hydroxylé de façon à séparer de la fraction prélevée à l'étape (b) un mélange contenant de l'eau et de la chlorhydrine présentant une teneur réduite en agent de chloration comparée à celle de la fraction prélevée à l'étape (b).

Dans le procédé de fabrication de l'épichlorhydrine selon l'invention, la séparation de la chlorhydrine et des autres composés du milieu réactionnel de chloration de l'hydrocarbure aliphatique polyhydroxylé peut être effectuée selon les modes tels que décrits dans la demande intitulée « Procédé de conversion d'hydrocarbures aliphatiques poly hydroxylés en chlorhydrines » déposée au nom de SOLVAY SA le même jour que la présente demande.

Mention particulière est faite d'un procédé de préparation d'une chlorhydrine comprenant les étapes suivantes : (a) on fait réagir un hydrocarbure aliphatique polyhydroxylé, un ester d'un hydrocarbure aliphatique polyhydroxylé , ou un mélange d'entre eux, avec un agent de chloration de façon à obtenir un mélange contenant de la chlorhydrine, des esters de chlorhydrine et de l'eau, (b) on soumet au moins une fraction du mélange obtenu à l'étape (a) à un traitement de distillation et/ou de stripping de façon à obtenir une partie concentrée en eau, en chlorhydrine et en esters de chlorhydrine, et (c) on soumet au moins une fraction de la partie obtenue à l'étape (b) à une opération de séparation en présence d'au moins un additif de façon à obtenir une portion concentrée en chlorhydrine et en esters de chlorhydrine et qui contient moins de 40 % en poids d'eau.

L'opération de séparation est plus particulièrement une décantation. Dans le procédé de fabrication de l'épichlorhydrine selon l'invention, la séparation et le traitement des autres composés du milieu réactionnel de chloration de l'hydrocarbure aliphatique polyhydroxylé peuvent être effectués selon des modes tels que décrits dans la demande intitulée « Procédé de fabrication d'une chlorhydrine par chloration d'un hydrocarbure aliphatique poly hydroxylé » déposée au nom de SOLVAY SA le même jour que la présente demande. Un traitement préféré consiste à soumettre une fraction des sous-produits de la réaction à une oxydation à haute température.

Mention particulière est faite d'un procédé de fabrication d'une chlorhydrine comprenant les étapes suivantes : (a) on fait réagir un hydrocarbure aliphatique poly hydroxylé, un ester d'un hydrocarbure aliphatique poly hydroxylé, ou un mélange d'entre eux, dont la teneur en métaux alcalins et/ou alcalino-terreux est inférieure ou égale à 5 /kg, un agent oxydant et un acide organique de façon à obtenir un mélange contenant au moins de la chlorhydrine et des sous-produits, (b) on soumet au moins une partie du mélange obtenu à l'étape (a) à un ou plusieurs traitements dans des étapes ultérieures à l'étape (a) et (c) au moins une des étapes ultérieures à l'étape (a) consiste en une oxydation a une température supérieure ou égale à 800°C.

Mention plus particulière est faite d'un procédé dans lequel dans l'étape ultérieure, on prélève une partie du mélange obtenu à l'étape (a) et on soumet cette partie à une oxydation à une température supérieure ou égale à 800°C, pendant le prélèvement. Mention particulière est aussi faite d'un procédé dans lequel le traitement de l'étape (b) est une opération de séparation choisie parmi les opérations de décantation, de filtration, de centrifugation, d'extraction, de lavage, d'évaporation, de stripping, de distillation, d'adsorption ou les combinaisons d'au moins deux d'entre-elles.

Dans le procédé selon l'invention, la chlorhydrine est le dichloropropanol, et celui-ci est généralement obtenu sous la forme d'un mélange de composés comprenant les isomères de 1,3-dichloropropane-2-ol et de 2,3-ichloropropane-1-ol. Ce mélange contient généralement plus de 1 % en poids des deux isomères, de préférence plus de 5 % en poids et de manière particulière plus de 50 %. Le mélange contient usuellement moins de 99,9 % en poids des deux isomères, de préférence moins de 95 % en poids et tout particulièrement moins de 90 % en poids. Les autres constituants du mélange peuvent être des composés provenant des procédés de fabrication du dichloropropanol, tels que des réactifs résiduels, des sous-produits de réaction, des solvants et notamment de l'eau.

Le rapport massique entre les isomères 1,3-dichloropropane-2-ol et 2,3-dichloropropane-1-ol est usuellement supérieur ou égal à 0,01, de préférence supérieur ou égal 0,4. Ce rapport est usuellement inférieur ou égal à 99 et de préférence inférieur ou égal à 25.

Dans le procédé selon l'invention, la chlorhydrine est le dichloropropanol et lorsque ce dernier est obtenu dans un procédé au départ de chlorure d'allyle, le mélange d'isomères présente un rapport massique 1,3-dichloropropane-2-ol : 2,3-dichloropropane-1-ol qui est souvent de 0,3 à 0,6, typiquement d'environ 0,5. Lorsque le dichloropropanol est obtenu dans un procédé au départ de glycérol synthétique et/ou naturel, le rapport massique 1 ,3 -dichloropropane-2-ol : 2,3 -dichloropropane-1-ol est habituellement supérieur ou égal à 1,5, souvent supérieur à ou égal à 3,0, fréquemment supérieur ou égal à 7,0 et tout particulièrement supérieur ou égal à 20,0. Lorsque le dichloropropanol est obtenu au départ d'alcool allylique, le rapport massique 1,3-dichloropropane-2-ol : 2,3-dichloro-propane-1-ol est souvent de l'ordre de 0,1.

Par cétones halogénées, on entend désigner les cétones comprenant de 3 à 18 atomes de carbone, de préférence de 3 à 12 atomes de carbone et de façon particulièrement préférée de 3 à 6 atomes de carbone, et dans lesquelles un ou plusieurs atomes d'hydrogène ont été remplacés par un atome d'halogène. Il s'agit souvent de cétones chlorées et plus particulièrement de la chloroacétone.

Ces cétones halogénées peuvent être produites dans certaines conditions lors de la déshydrochloration de la chlorhydrine et ou préalablement à celle-ci, dans les procédés de fabrication de la chlorhydrine. Dans le premier cas, la chlorhydrine étant le dichloropropanol, et sans vouloir être lié par une quelconque théorie, on pense que la chloroacétone est essentiellement générée au départ de l'isomère 1,3-dichloropropane-2-ol. Dans le second cas, on a découvert de façon surprenante que les cétones halogénées peuvent être présentes en grande quantité dans la chlorhydrine obtenue par un procédé d'hydrochloration d'un hydrocarbure aliphatique poly hydroxylé. Dans ce cas, la teneur de la chlorhydrine obtenue en cétones halogénées est habituellement supérieure ou égale à 0,005 % en poids par rapport au mélange des isomères de la chlorhydrine et souvent supérieure ou égale à 0,01 % en poids. Cette teneur est habituellement inférieure ou égale à 0,4 % en poids par rapport au mélange des isomères de la chlorhydrine et de préférence inférieure ou égale à 0,3 % en poids.

Selon une première variante du procédé selon l'invention, le traitement destiné à éliminer au moins une partie de la chloroacétone est effectué pendant la déshydrochloration de la chlorhydrine.

Par déshydrochloration, on entend désigner l'élimination d'acide chlorhydrique, quel que soit le mécanisme de cette élimination et la forme ultime sous laquelle se retrouve l'acide chlorhydrique éliminé.

La déshydrochloration peut être effectuée par tout moyen connu, par exemple par chauffage de la chlorhydrine en absence de réactif autre que la chlorhydrine, par traitement de la chlorhydrine par un composé basique, en présence ou non d'un catalyseur. On préfère effectuer la déshydrochloration en traitant la chlorhydrine par un composé basique.

Par composé basique, on entend désigner des composés organiques basiques ou des composés inorganiques basiques. Les composés inorganiques basiques sont préférés. Ces composés inorganiques basiques peuvent être des oxydes, des hydroxydes et des sels de métaux, comme des carbonates, des hydrogénocarbonates, des phosphates ou leurs mélanges, par exemple. Parmi les métaux, les métaux alcalins et alcalino-terreux sont préférés. Le sodium, le potassium et le calcium et leurs mélanges sont particulièrement préférés. Les composés inorganiques basiques peuvent se présenter sous la forme de solides, de liquides, de solutions ou de suspensions aqueuses or organiques. Les solutions ou les suspensions aqueuses sont préférées. Les solutions et suspensions de NaOH, de Ca(OH)2, la saumure alcaline épurée et leurs mélanges sont particulièrement préférés. Par saumure alcaline épurée, on entend désigner de la soude caustique qui contient du NaCl telle que celle produite dans un procédé d'électrolyse à diaphragme. La teneur en composé basique dans la solution ou la suspension est généralement supérieure ou égale à 1 % en poids, de préférence supérieure ou égale à 4 % en poids et de façon tout particulièrement préférée supérieure ou égale à 6 % en poids. Cette teneur est habituellement inférieure ou égale à 60 % en poids. Une teneur d'environ 50 % en poids convient particulièrement bien.

Le composé basique peut être utilise en quantités sur-stoechiométriques, sous-stoechiométriques ou stoechiométriques par rapport à la chlorhydrine. Lorsque le composé basique est utilisé en quantités sous-stoechiométriques, on utilise généralement au plus 2 moles de chlorhydrine par mole de base. On utilise souvent au plus 1,5 mole de chlorhydrine par mole de base et de préférence au plus 1,05 mole de chlorhydrine par mole de base. Lorsque l'agent basique est utilisé en quantités sur-stoechiométriques, on utilise au plus 2 mole de base par mole de chlorhydrine. Dans ce cas, on utilise généralement au moins de 1,05 mole de base par mole de chlorhydrine.

La teneur en eau du mélange comprenant la chlorhydrine et le composé basique est généralement supérieure à 8 % en poids.

Lorsque la déshydrochloration est réalisée par traitement de la chlorhydrine par un composé basique, le milieu de réaction peut aussi contenir un solvant tels que ceux décrits dans le brevet US 3,061,615 au nom de SOLVAY SA

La déshydrochloration peut être effectuée comme décrit décrits dans la demande intitulée « Procédé de fabrication d'un époxyde au départ d'un hydrocarbure aliphatique poly hydroxylé et d'un agent de chloration » déposée au nom de SOLVAY SA le même jour que la présente demande.

Mention particulière est faite d'un procédé de fabrication d'épichlorhydrine dans lequel on soumet un milieu réactionnel résultant de la réaction entre un hydrocarbure aliphatique poly hydroxylé, un ester d'un hydrocarbure aliphatique poly hydroxylé, ou un mélange d'entre eux, et un agent de chloration, le milieu réactionnel contenant au moins 10 g de chlorhydrine par kg de milieu réactionnel, à une réaction chimique ultérieure sans traitement intermédiaire.

Mention est également faite de fabrication d'épichlorhydrine comprenant les étapes suivantes : (a) on fait réagir un hydrocarbure aliphatique poly hydroxylé, un ester d'un hydrocarbure aliphatique poly hydroxylé, ou un mélange d'entre eux, avec un agent de chloration et un acide organique de façon à former de la chlorhydrine et des esters de chlorhydrine dans un milieu réactionnel contenant de l'hydrocarbure aliphatique poly hydroxylé, de l'ester d'hydrocarbure aliphatique poly hydroxylé, de l'eau, l'agent de chloration et l'acide organique, le milieu réactionnel contenant au moins 10 g de chlorhydrine par kg de milieu réactionnel, (b) on soumet au moins une fraction du milieu réactionnel obtenu à l'étape (a), fraction qui à la même composition que le milieu réactionnel obtenu à l'étape (a), à un ou plusieurs traitements dans des étapes ultérieures à l'étape (a), et (c) on ajoute un composé basique 51 au moins une des étapes ultérieures à l'étape (a) pour qu'il réagisse au moins partiellement avec la chlorhydrine, les esters de chlorhydrine, l'agent de chloration et l'acide organique de façon à former de l'épichlorhydrine et des sels.

Le milieu liquide de réaction peut être monophasique ou biphasique.

Le traitement basique de déshydrochloration peut être mené en mode continu ou discontinu.

La durée du traitement basique ou le cas échéant, le temps de séjour des réactifs lors du traitement basique est généralement supérieur ou égal à 0,1 s, de préférence supérieur ou égal à 0,3 s et de façon particulièrement préférée supérieur ou égal à 0,4 s. Cette durée ou ce temps de séjour est habituellement inférieur ou égal à 2 h, plus spécialement inférieur ou égal à 1 h. Le temps de séjour des réactifs est défini comme le rapport entre le volume du réacteur occupé par la ou les phases liquides et le débit cumulé des réactifs.

Le traitement basique de déshydrochloration selon l'invention est généralement effectué à une température d'au moins 0°C. Souvent cette température est d'au moins 20°C. De préférence, elle est d'au moins 30°C. Dans le procédé de fabrication de l'épichlorhydrine selon l'invention, la réaction est généralement effectuée à une température d'au plus 140°C. De préférence elle est d'au plus 120°C. Dans une première variante particulière, la température est de 25 à 50°C. Dans une deuxième variante particulière, la température est de 50 à 90°C.

Dans le procédé de fabrication de l'épichlorhydrine selon l'invention, la déshydrochloration est généralement effectuée à une pression d'au moins 0,08 bar absolu. Souvent cette pression est d'au moins 0,1 bar absolu. De préférence elle est d'au moins 0,2 bar absolu. Dans le procédé de fabrication de l'épichlorhydrine selon l'invention, la déshydrochloration est généralement effectuée à une pression d'au plus 25 bar absolu. De préférence elle est d'au plus 6 bar absolu. Dans une première variante particulière, la pression est de 0,4 51 0,8 bar absolu. Dans une deuxième variante particulière, la pression est de 1 à 3 bar. L'épichlorhydrine qui est formée dans le procédé de déshydrochloration de la chlorhydrine, peut être éliminé au fur et à mesure de sa formation, par distillation ou stripping. Le stripping peut être effectué au moyen de n'importe quel gaz inerte vis-à-vis de l'épichlorhydrine. On préfère effectuer ce stripping à la vapeur d'eau.

Après traitement basique, la teneur en cétones halogénées de l'épichlorhydrine est habituellement inférieure ou égale à 0,01 % en poids, de préférence inférieure ou égale à 0,005 % en poids et de manière tout particulièrement préférée inférieure ou égale à 0,003 % en poids. Souvent, l'épichlorhydrine contient au moins 0,0001 % en poids de cétones halogénées.

Est également décrit de l'épichlorhydrine dont la teneur en cétones halogénées est inférieure ou égale à 0,01 % en poids. La pureté de l'épichlorhydrine est de préférence supérieure ou égale à 999 g/kg.

Sans vouloir être lié par une quelconque théorie, on pense que les réactivités de la chlorhydrine, des cétones halogénées et de l'épichlorhydrine sont telles qu'il est possible d'éliminer les cétones halogénées sans affecter le rendement en épichlorhydrine en choisissant judicieusement les conditions de déshydrochloration de la chlorhydrine.

Selon une seconde variante du procédé selon l'invention, le traitement destiné à éliminer au moins une partie de la chloroacétone comprend une distillation, une évaporation et/ou un stripping en présence d'eau, ce traitement est effectué préalablement à la déshydrochloration, et il permet d'éliminer une fraction constituée essentiellement d'eau et de chloroacétone, et de récupérer de la chlorhydrine présentant une teneur réduite en chloroacétone.

Après ce traitement, la teneur en chloroacétone de la chlorhydrine est habituellement inférieure ou égale à 0,1 % en poids par rapport au mélange des isomères de la chlorhydrine, de préférence inférieure ou égale à 0,04 % en poids et de manière tout particulièrement préférée inférieure ou égale à 0.005 % en poids. Souvent, la chlorhydrine contient au moins 0,0001 % en poids de cétones halogénées par rapport au mélange au mélange des isomères de la chlorhydrine.

Ce traitement est de préférence une distillation azéotropique en présence d'eau. On a en effet découvert que lorsque la cétone halogénée est la chloroacétone par exemple, l'eau et la chloroacétone forment un mélange azéotropique binaire à point bas, dont la composition peut être caractérisée par sa température d'ébullition qui est de 92°C à 1013 mbar. Cette composition est constituée, à cette température et pression de 28 % en poids d'eau et de 72 % en poids de chloroacétone. Deux phases liquides se séparent après condensation à 25°C ; la phase organique plus dense contient 95 % en poids de chloroacétone et 5 % en poids d'eau tandis que la phase aqueuse contient 5 % en poids de chloroacétone et 95 % en poids d'eau. On a découvert que l'exploitation des propriétés des équilibres liquide-vapeur de la composition binaire eau chloroacétone permettait d'enlever la chloroacétone du dichloropropanol. L'eau nécessaire à la distillation azéotropique peut provenir par exemple d'un procédé de synthèse du dichloropropanol, en particulier par chloration du glycérol ou être amenée ultérieurement dans le procédé.

Est aussi décrit une composition azéotropique comprenant de l'eau et de la chloroacétone.

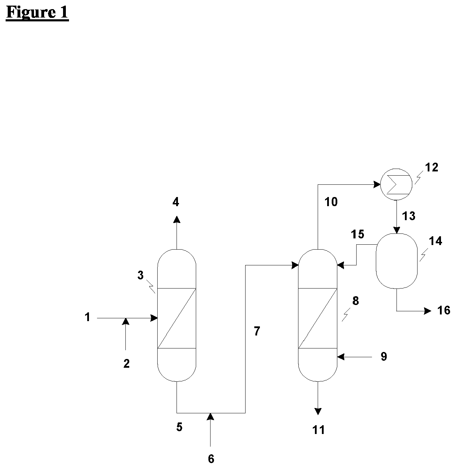

La figure 1 montre un premier schéma particulier d'une installation qui peut être utilisée pour conduire le procédé de fabrication de l'épichlorhydrine selon l'invention.

Une colonne de distillation (3) est alimentée via la ligne (1) avec de la chlorhydrine. De l'eau est ajoutée à la chlorhydrine via la ligne (2). Un flux qui contient de l'eau et la majeure partie des cétones halogénées est continuellement soutiré de la colonne (3) via la ligne (4). Le résidu de la colonne contenant la chlorhydrine épurée est soutiré via la ligne (5). Un composé basique est ajouté au résidu de la colonne (3) via la ligne (6) et le mélange obtenu alimente un réacteur pouvant éventuellement servir de colonne de distillation (8) via la ligne (7). De la vapeur est introduite dans le pied du réacteur (8) via la ligne (9). Un flux gazeux est continuellement soutiré du réacteur (8) via la ligne (10) et alimente un condenseur (12). Un flux liquide est continuellement soutiré du réacteur (8) via la ligne (11). Le flux condensé (13) alimente un décanteur (14). La phase aqueuse décantée est renvoyée dans le haut du réacteur (8) par la canalisation (15) afin d'assurer un reflux. L'épichlorhydrine brute produite constitue la phase organique décantée qui est soutirée par la canalisation (16). Cette épichlorhydrine brute est purifiée dans un secteur de distillation.

Dans un premier aspect particulier du procédé selon l'invention, on met en oeuvre de la chlorhydrine contenant au moins une partie de la chlorhydrine issue d'une fabrication au départ d'un hydrocarbure aliphatique poly hydroxylé par réaction avec un agent de chloration. Dans cet aspect, la partie de la chlorhydrine issue d'une fabrication au départ de l'hydrocarbure aliphatique poly hydroxylé par réaction avec un agent de chloration, constitue généralement au moins 1 % en poids par rapport au poids total de la chlorhydrine, de préférence au moins 5 % en poids, et plus particulièrement au moins 35 % en poids. Dans cet aspect particulier, cette fraction est généralement d'au plus 99 % en poids et de préférence d'au plus 60 % en poids. L'appoint de la chlorhydrine pouvant étre issu de l'un quelconque des autres procédés de fabrication du la chlorhydrine envisages ci-dessus est généralement d'au moins de 1 % en poids, de préférence d'au moins 5 % en poids, et plus particulièrement d'au moins 35 % en poids. Cette fraction est d'au plus 99 % en poids et de préférence d'au plus 60 % en poids. Parmi ces autres procédés de fabrication de la chlorhydrine, l'hypochloration d'une oléfine est préférée.

L'invention concerne dès lors aussi un premier procédé de fabrication d'épichlorhydrine comprenant :
(a) une étape de fabrication d'une chlorhydrine par hypochloration d'une oléfine
(b) une étape de fabrication de la chlorhydrine par chloration d'un hydrocarbure aliphatique poly hydroxylé, effectuée en parallèle avec l'étape (a), et
(c) une étape commune de déshydrochloration dans laquelle on met en oeuvre de la chlorhydrine obtenue selon les étapes (a) et (b).
De préférence, ce premier procédé de fabrication de l'épichlorhydrine comprend une étape de traitement destinée à éliminer au moins une partie de la chloroacétone formée dans le procédé.

Selon un premier mode de réalisation du premier procédé de fabrication de l'épichlorhydrine selon l'invention, on met en oeuvre à l'étape (c) un mélange de la chlorhydrine obtenue selon les étapes (a) et (b).

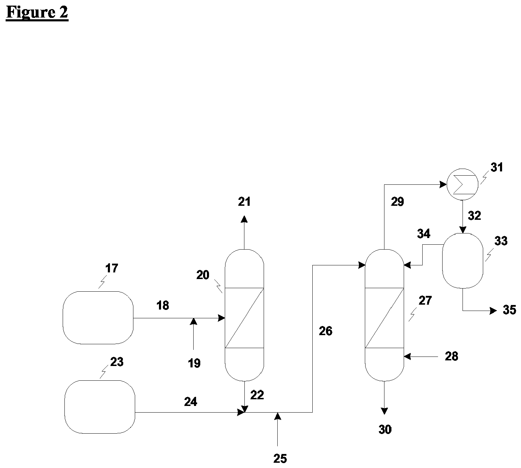

La figure 2 montre un deuxième schéma particulier d'une installation qui peut être utilisée pour conduire le procédé de fabrication d'une épichlorhydrine selon ce mode de réalisation.

Une colonne de distillation (20) est alimentée via la ligne (18) avec de la chlorhydrine provenant d'un réacteur de chloration d'un hydrocarbure aliphatique poly hydroxylé (17). De l'eau est ajoutée à la chlorhydrine via la ligne (19). Un flux qui contient de l'eau et des cétones halogénées est continuellement soutiré de la colonne (20) via la ligne (21). Le résidu de la colonne est soutiré via la ligne (22) et est mélangé à un flux de la chlorhydrine (24) issu d'un réacteur d'hypochloration d'une oléfine (23). Un composé basique est ajouté au flux mixte de la chlorhydrine via la ligne (25) et le mélange obtenu alimente un réacteur pouvant éventuellement servir de colonne de distillation (27) via la ligne (26). De la vapeur est introduite dans le pied du réacteur (27) via la ligne (28). Un flux gazeux est continuellement soutiré du réacteur (27) via la ligne (29) et alimente un condenseur (31). Un flux liquide est continuellement soutiré du réacteur (27) via la ligne (30). Le flux condense (32) alimente un décanteur (33). La phase aqueuse décantée est renvoyée dans le haut du réacteur (27) par la canalisation (34) afin d'assurer un reflux. L'épichlorhydrine brute produite constitue la phase organique décantée qui est soutirée par la canalisation (35). Cette épichlorhydrine brute est purifiée dans un secteur de distillation.

Selon un deuxième mode de réalisation du premier procédé de fabrication de l'épichlorhydrine selon l'invention, on met en oeuvre à l'étape (c) alternativement ou simultanément de la chlorhydrine obtenue selon l'étape (a) ou selon l'étape (b).

Est également décrit une installation de fabrication d'une l'épichlorhydrine comprenant :
(a) un réacteur d'hypochloration d'une oléfine duquel sort un milieu réactionnel contenant une chlorhydrine
(b) un réacteur de chloration d'un hydrocarbure aliphatique poly hydroxylé duquel sort un milieu réactionnel contenant de la chlorhydrine, et
(c) un réacteur de déshydrochloration alimente par les milieux réactionnels sortant du réacteur (a) et du réacteur (b).

Cette installation de fabrication de l'épichlorhydrine comprend de préférence un réacteur pour le traitement d'élimination d'au moins une partie des cétones halogénées formées dans l'installation.

Les conditions de l'étape (b) peuvent être celles décrites dans la demande WO 2005/054167 de SOLVAY SA et dans la demande EP 05104321.4 déposée au nom de SOLVAY SA le 20/05/2005. Les conditions de l'étape (c) sont, par exemple, comme décrites plus haut dans la présente demande.

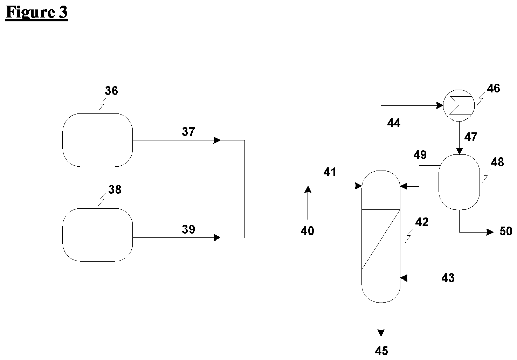

La figure 3 montre un troisième schéma particulier d'une installation qui peut être utilisée pour conduire le procédé de fabrication de l'épichlorhydrine selon l'invention.

Un flux de chlorhydrine (37) venant d'un réacteur de chloration d'un hydrocarbure aliphatique poly hydroxylé (36) est mélangé à un flux de la chlorhydrine (39) issu d'un réacteur d'hypochloration d'une oléfine (38). Un composé basique est ajouté au flux mixte de chlorhydrine via la ligne (40) et le mélange obtenu alimente un réacteur pouvant éventuellement servir de colonne de distillation (42) Via la ligne (41). De la vapeur est introduite dans le pied du réacteur (42) Via la ligne (43). Un flux gazeux est continuellement soutiré du réacteur (42) Via la ligne (44) et alimente un condenseur (46). Un flux liquide est continuellement soutiré du réacteur (42) via la ligne (45). Le flux condense (47) alimente un décanteur (48). La phase aqueuse décantée est renvoyée dans le haut réacteur (42) par la canalisation (49) afin d'assurer un reflux. L'épichlorhydrine brute produite constitue la phase organique décantée qui est soutirée par la canalisation (50). Cette épichlorhydrine brute est purifiée dans un secteur de distillation.

Dans un deuxième aspect particulier du procédé selon l'invention, on obtient de l'épichlorhydrine dont une partie au moins est issue d'une fabrication séparée de l'épichlorhydrine au départ d'une chlorhydrine obtenue par réaction d'un hydrocarbure aliphatique poly hydroxylé avec un agent de chloration. Dans cet aspect, la partie de la chlorhydrine issue d'une fabrication au départ de l'hydrocarbure aliphatique chloré par réaction avec un agent de chloration, constitue généralement au moins 1 % en poids par rapport au poids total de l'épichlorhydrine, de préférence au moins 5 % en poids, et plus particulièrement au moins 35 % en poids. Dans cet aspect particulier, cette fraction est généralement d'au plus 99 % en poids et de préférence d'au plus 60 % en poids. L'appoint de l'épichlorhydrine peut être issu de l'un quelconque des autres procédés de fabrication de la chlorhydrine envisagés ci-dessus et est généralement d'au moins de 1 % en poids, de préférence d'au moins 5 % en poids, et plus particulièrement d'au moins 35 % en poids. Cette fraction est d'au plus 99 % en poids et de préférence d'au plus 60 % en poids.

Parmi ces autres procédés de fabrication de la chlorhydrine, l'hypochloration d'une oléfine est préférée.

L'invention concerne dès lors aussi un second procédé de fabrication l'épichlorhydrine comprenant :
(a) une étape de fabrication d'une chlorhydrine par hypochloration d'une oléfine
(b) une étape de déshydrochloration de la chlorhydrine obtenue selon l'étape (a) de façon à obtenir de l'épichlorhydrine dans un milieu réactionnel de déshydrochloration,
(c) une étape de fabrication de la chlorhydrine par chloration d'un hydrocarbure aliphatique poly hydroxylé
(d) une étape de déshydrochloration de la chlorhydrine obtenue selon l'étape (c) de façon à obtenir de l'épichlorhydrine dans un milieu réactionnel de déshydrochloration, et
(e) une étape de séparation de l'épichlorhydrine des milieux réactionnels de déshydrochloration dans laquelle on met en oeuvre le milieu réactionnel de déshydrochloration contenant de l'épichlorhydrine obtenue selon les étapes (b) et (d),
et dans lequel, l'étape (b) est consécutive à l'étape (a), l'étape (d) est consécutive à l'étape (c), et le couple constitué des étapes (a) et (b) est parallèle au couple constitué des étapes (c) et (d).

De préférence, ce second procédé de fabrication de l'épichlorhydrine comprend une étape de traitement destinée à éliminer au moins une partie des cétones halogénées formées dans le procédé.

Selon un premier mode de réalisation du second procédé de fabrication de l'épichlorhydrine selon l'invention, on met en oeuvre à l'étape (e) un mélange de l'épichlorhydrine obtenue selon les étapes (b) et (d).

Selon un deuxième mode de réalisation du deuxième procédé de fabrication de l'épichlorhydrine selon l'invention, on met en oeuvre à l'étape (e) alternativement ou simultanément de l'épichlorhydrine obtenue selon l'étape (b) ou selon l'étape (d).

Est également décrite une installation de fabrication
d'épichlorhydrine comprenant :
(a) un réacteur d'hypochloration d'une oléfine duquel sort un milieu réactionnel contenant une chlorhydrine
(b) un réacteur de déshydrochloration alimenté par le milieu réactionnel sortant du réacteur (a) duquel sort un milieu réactionnel contenant de l'épichlorhydrine
(c) un réacteur de chloration d'un hydrocarbure aliphatique polyhydroxylé duquel sort un milieu réactionnel contenant de la chlorhydrine
(d) un réacteur de déshydrochloration alimenté par le milieu réactionnel sortant du réacteur (b), duquel sort un milieu réactionnel contenant de l'épichlorhydrine et
(e) un séparateur alimenté(e) par les milieux réactionnels sortant du réacteur (b) et du réacteur (d)
et dans laquelle, le réacteur (b) est consécutif au réacteur (a), le réacteur (d) est consécutif au réacteur (c), et le couple constitué par les réacteurs (a) et (b) est parallèle au couple constitué par les réacteurs (c) et (d).

Cette installation de fabrication d'épichlorhydrine comprend de préférence un réacteur pour le traitement d'élimination d'au moins une partie des cétones
halogénées formées dans l'installation.

Les conditions de l'étape (c) peuvent être celles décrites dans la demande WO 2005/054167 au nom de SOLVAY SA et dans la demande EP 05104321.4. déposée au nom de SOLVAY SA le 20 mai 2005. Les conditions des étapes (c) et (d) sont, par exemple, comme décrites plus haut, dans la présente demande.

Le procédé de fabrication de l'épichlorhydrine selon l'invention peut être intégré dans un schéma global tel que décrit dans la demande intitulée « Procédé de fabrication d'un époxyde au départ d'une chlorhydrine » déposée au nom de SOLVAY SA le même jour que la présente demande,.

Mention particulière est faite d'un procédé de fabrication de l'épichlorhydrine comprenant au moins une étape de purification de l'épichlorhydrine formée, l'épichlorhydrine étant au moins en partie fabriqué par un procédé de déshydrochloration d'une chlorhydrine, celle-ci étant au moins en partie fabriquée par un procédé de chloration d'un hydrocarbure aliphatique poly hydroxylé, d'un ester d'un hydrocarbure aliphatique poly hydroxylé, ou d'un mélange d'entre eux.

Il a été prouvé qu'il est possible d'augmenter de maniéré économique la capacité d'installations de fabrication d'épichlorhydrine au départ de matières premières fossiles sans augmentation de consommation de ces matières premières.

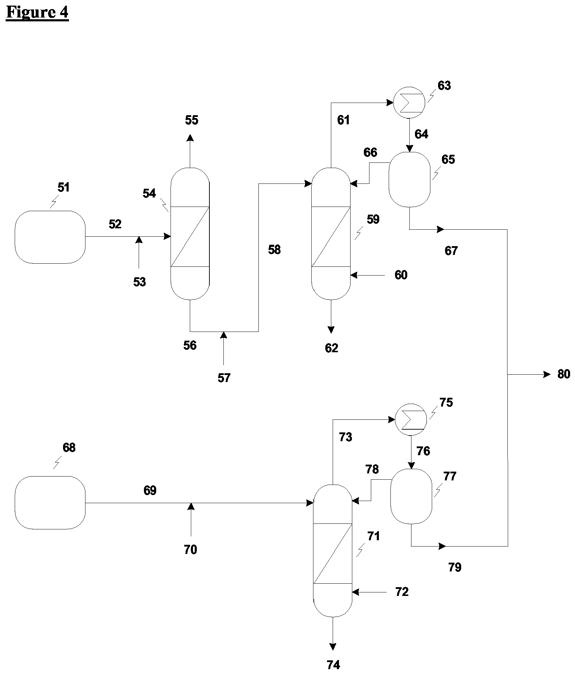

La figure 4 montre un quatrième schéma particulier d'une installation qui peut être utilisée pour conduire le procédé de fabrication de l'épichlorhydrine selon l'invention.

Une colonne de distillation (54) est alimentée en chlorhydrine provenant d'un réacteur de chloration d'un hydrocarbure aliphatique poly hydroxylé (51) via la ligne (52). De l'eau est ajoutée à la chlorhydrine via la ligne (53). Un flux qui contient de l'eau et des cétones halogénée est continuellement soutiré de la colonne (54) via la ligne (55). Le résidu de la colonne (54) est soutiré via la ligne (56). Un composé basique est ajouté au résidu de la colonne (54) via la ligne (57) et le mélange obtenu alimente un réacteur pouvant éventuellement servir de colonne de distillation (59) via la ligne (5 8). De la vapeur est introduite dans le pied du réacteur (59) via la ligne (60). Un flux gazeux est continuellement soutiré du réacteur (59) via la ligne (61) et alimente un condenseur (63). Un flux liquide est continuellement soutiré du réacteur (59) via la ligne (62). Le flux condense (64) alimente un décanteur (65). La phase aqueuse décantée est renvoyée dans le haut du réacteur (59) par la canalisation (66) afin d'assurer un reflux. L'épichlorhydrine brute produite constitue la phase organique décantée qui est soutirée par la canalisation (67).

Un autre un réacteur pouvant éventuellement servir de colonne de distillation (71) est alimente en chlorhydrine provenant d'un réacteur d'hypochloration d'une oléfine (68) via la ligne (69) et un composé basique est ajouté à la chlorhydrine via la ligne (70). De la vapeur est introduite dans le pied du réacteur (71) via la ligne (72). Un flux gazeux est continuellement soutiré du réacteur (71) via la ligne (73) et alimente un condenseur (75). Un flux liquide est continuellement soutiré du réacteur (71) via la ligne (74). Le flux condense (76) alimente un décanteur (77). La phase aqueuse décantée est renvoyée dans le haut du réacteur (71) par la canalisation (78) afin d'assurer un reflux. L'épichlorhydrine brute produite constitue la phase organique décantée qui est soutirée par la canalisation (79). Les deux flux de l'épichlorhydrine brute sont rassemblés préalablement en vue d'une purification dans un secteur de distillation commun via la ligne (80).

Dans les procédés et installations décrits ci-dessus, la chlorhydrine est le dichloropropanol, l'hydrocarbure aliphatique poly hydroxylé est le glycérol et l'oléfine est le chlorure d'allyle.

Les procédés et installations de fabrication de l'épichlorhydrine peuvent être suivis de procédés et d'installations de fabrication de résines époxy.

Les exemples ci-après entendent illustrer l'invention sans toutefois la limiter.

### Exemple 1

On additionne 84 g de soude aqueuse concentrée à 50 % en poids (1,05 mol) à une solution de 129 g de 1,3-dichloropropan-2-ol dans 950 ml d'eau. Le dichloropropanol contient 3 g/kg de chloroacétone. Après 1 minute de réaction à température ambiante, la conversion du 1,3-dichloro-propan-2-ol est complète, la sélectivité en épichlorhydrine est de 99,9 % et la teneur relative en chloroacétone par rapport à l' épichlorhydrine produite est réduite à 11 mg/kg. La sélectivité en produits d'hydrolyse d'épichlorhydrine est de 0,1 %.

### Exemple 2

Un mélange de 434,6 g de 1,3-dichloropropan-2-ol contenant 3,7 g/kg de chloroacétone a été distillé après addition de 66,5 g d'eau.

La distillation a été réalisée à pression atmosphérique au moyen d'une colonne adiabatique à plateaux surmontée d'un dispositif permettant de refluer en haut de colonne une partie de la phase vapeur. Le taux de reflux en haut de colonne a été fixé à 57 %.

Le tableau de résultat ci-dessous décrit la composition des différentes fractions collectées :

| Fraction | Température Tête de colonne | Masse | Chloroacétone | 1,3 dichloro-Propan-2-ol | Eau |
|---|---|---|---|---|---|
| | °C | g | g/kg | g/kg | g/kg |
| Mélange de départ | | 488,7 | 3,2 | 864,2 | 132,6 |
| Fraction distillée 1 | 94 | 1,77 | 56 | 181 | 762 |
| Fraction distillée 2 | 99 | 3,54 | 32 | 231 | 737 |
| Fraction distillée 3 | 99 | 3,33 | 25 | 241 | 734 |
| Fraction distillée 4 | 98 | 7,87 | 23 | 283 | 695 |
| Fraction distillée 5 | 95 | 11,62 | 26 | 261 | 713 |
| Fraction distillée 6 | 97 | 12,3 | 25 | 227 | 749 |
| Résidu dans bouilleur | | 433,80 | 1 | 927 | 72 |

On a récolté 40,43 g de distillat en 6 fractions et chaque fraction de distillat était biphasique. Les concentrations données dans le tableau se rapportent à la somme des deux phases. Cette distillation a permis d'éliminer 60 % de la chloroacétone initialement présente avec une perte totale limitée à 1,8 % de 1,3-dichloropropan-2-ol dans les distillats.

## Revendications

1. Procédé de fabrication d'épichlorhydrine par déshydrochloration de dichloropropanol, dans lequel au moins une fraction du dichloropropanol est fabriquée par chloration de glycérol, et dans lequel de la chloroacétone est formée comme sous-produit et qui comprend au moins un traitement d'élimination d'au moins une partie de la chloroacétone formée.

2. Procédé selon la revendication 1 dans lequel le glycérol est obtenu au départ de matières premières renouvelables.

3. Procédé selon la revendication 1 ou 2, dans lequel le traitement destiné à éliminer au moins une partie de la chloroacétone est effectué pendant la déshydrochloration.

4. Procédé selon l'une quelconque des revendications 1 à 3, dans lequel la déshydrochloration est effectuée en traitant le dichloropropanol par un composé basique, et où le compose basique est sélectionné parmi les solutions ou les suspensions aqueuses de NaOH, de Ca(OH)₂, la saumure alcaline épurée et leurs mélanges.

5. Procédé selon l'une quelconque des revendications 1 à 4, dans lequel la déshydrochloration est effectuée en mode discontinu ou en mode continu, pendant une durée ou un temps de séjour des réactifs supérieur ou égal à 0,1 s et inférieur ou égal à 2 h et à une température d'au moins 0°C et d'au plus 140°C, à une pression d'au moins 0,8 bar absolu et d'au plus 25 bar absolu.

6. Procédé selon l'une quelconque des revendications 1 à 5 dans lequel le glycérol est obtenu au cours de la fabrication de biodiesel, ou au cours de transformations de graisses et huiles, d'origine végétale ou animale, les transformations étant sélectionnées parmi les réactions de saponification, de trans-estérification et d'hydrolyse.

7. Procédé selon la revendication 6 dans lequel la réaction de trans-estérification est réalisée en présence d'un catalyseur hétérogène.

## Patentansprüche

1. Verfahren zum Herstellen von Ephichlorhydrin durch Dehydrochlorierung von Dichlorpropanol, wobei mindestens ein Anteil des Dichlorpropanols durch Chlorieren von Glycerin hergestellt wird, und wobei Chloraceton als Nebenprodukt gebildet wird, und das mindestens eine Behandlung zum Eliminieren von mindestens einem Teil des gebildeten Chloracetons aufweist.

2. Verfahren gemäß Anspruch 1, wobei das Glycerin aus erneuerbaren Rohmaterialien erhalten wird.

3. Verfahren gemäß Anspruch 1 oder 2, wobei die Behandlung, die zum Eliminieren von mindestens einem Teil des Chloracetons vorgesehen ist, während der Dehydrochlorierung durchgeführt wird.

4. Verfahren gemäß irgendeinem der Ansprüche 1 bis 3, wobei die Dehydrochlorierung durchgeführt wird durch Behandeln des Dichlorpropanols mit einer basischen Verbindung, und wobei die basische Verbindung aus wässrigen Lösungen oder Suspensionen von NaOH, Ca(OH)₂, gereinigter alkalischer Salzlake und deren Mischungen ausgewählt ist.

5. Verfahren gemäß irgendeinem der Ansprüche 1 bis 4, wobei die Dehydrochlorierung diskontinuierlich oder kontinuierlich während einer Dauer oder einer Verweildauer der Reagenzien von größer oder gleich 0,1 s und kleiner oder gleich 2 h und bei einer Temperatur von mindestens 0°C und höchstens 140°C, bei einem Druck von mindestens 0,8 bar absolut und höchstens 25 bar absolut durchgeführt wird.

6. Verfahren gemäß irgendeinem der Ansprüche 1 bis 5, wobei das Glycerin während der Herstellung von Biodiesel oder während Umwandlungen von Fetten und Ölen pflanzlichen oder tierischen Ursprungs erhalten wird, wobei die Umwandlungen aus den Reaktionen der Verseifung, der Umesterung und der Hydrolyse ausgewählt sind.

7. Verfahren gemäß Anspruch 6, wobei die Reaktion der Umesterung in Anwesenheit eines heterogenen Katalysators erfolgt.

## Claims

1. Process for the production of epichlorohydrin by dehydrochlorination of dichloropropanol, wherein at least one fraction of the dichloropropanol is produced by chlorination of glycerol and wherein chloroacetone is formed as by-product and which comprises at least one treatment of removal of at least a portion of the chloroacetone formed.

2. Process according to claim 1, wherein the glycerol is obtained starting from renewable raw materials.

3. Process according to claim 1 or 2, wherein the treatment for removing at least a portion of the chloroacetone is carried out during the dehydrochlorination.

4. Process according to any one of claims 1 to 3, wherein the dehydrochlorination is carried out by treating the dichloropropanol with a basic compound, and wherein the basic compound is selected from aqueous solutions or suspensions of NaOH, of Ca(OH)2, purified alkaline brine and mixtures thereof.

5. Process according to any one of claims 1 to 4, wherein the dehydrochlorination is carried out discontinuously or continuously for a period or a dwell time of the reagents of greater than or equal to 0.1 second and less than or equal to 2 hours and at a temperature of at least 0°C and not more than 140°C, at a pressure of at least 0.8 bar absolute and not more than 25 bar absolute.

6. Process according to any one of claims 1 to 5, wherein the glycerol is obtained during the manufacture of biodiesel or during conversions of fats and oils of plant or animal origin, the conversions being selected from saponification, transesterification and hydrolysis reactions.

7. Process according to claim 6, wherein the transesterification reaction is carried out in the presence of a heterogeneous catalyst.
